(19) Europäisches Patentamt
European Patent Office
Office européen des brevets

(11) Veröffentlichungsnummer: **0 104 483**
**A2**

(12) # EUROPÄISCHE PATENTANMELDUNG

(21) Anmeldenummer: 83108577.4

(22) Anmeldetag: 31.08.83

(51) Int. Cl.³: C 07 C 143/78
A 61 K 31/255

(30) Priorität: 04.09.82 DE 3232922

(43) Veröffentlichungstag der Anmeldung: 04.04.84 Patentblatt 84/14

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Seuring, Bernhard, Dr.
Frankfurter Strasse 19
D-6238 Hofheim am Taunus(DE)

(72) Erfinder: Lang, Hans-Jochen, Dr.
Rüdesheimer Strasse 7
D-6238 Hofheim am Taunus(DE)

(72) Erfinder: Hropot, Max, Dr.
Friedrich-Stolz-Strasse 13
D-6093 Flörsheim am Main(DE)

(72) Erfinder: Muschaweck, Roman, Dr.
Heimchenweg 39
D-6230 Frankfurt am Main 80(DE)

(54) **Sulfamoylbenzophenon-Derivate, Verfahren zu ihrer Herstellung, ihre Verwendung sowie pharmazeutische Präparate auf Basis dieser Verbindungen.**

(57) Verbindungen der Formel

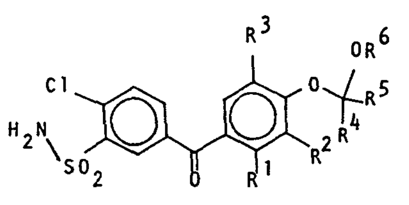

(I)

in welcher
$R^1$, $R^2$ und $R^3$ gleich oder verschieden sind und für Wasserstoff, Halogen, Alkyl mit 1 bis 4 C-Atomen oder Alkoxy mit 1 bis 4 C-Atomen stehen, wobei aber $R^1$ und $R^2$ nicht gleichzeitig Wasserstoff bedeuten können und wobei $R^1$ und $R^2$ auch gemeinsam eine Brücke aus zwei bis 5 Methylengruppen oder Methylendioxy bedeuten können,
$R^4$ und $R^5$ gleich oder verschieden sind und für Wasserstoff oder Alkyl mit 1 bis 4 C-Atomen stehen oder Bestandteil eines 3- bis 7-gliedrigen gesättigten carbocyclischen Ringes sind und
$R^6$ für Wasserstoff, Alkyl mit 1 bis 4 C-Atomen, die gegebenenfalls mit Hydroxy oder Alkoxy mit 1 bis 3 C-Atomen substituiert sein können, oder Aralkyl mit 7 bis 9 C-Atomen steht, bedeutet
sowie deren physiologisch verträgliche Salze über urikosurische und salidiuretische Wirkung aus.

EP 0 104 483 A2

Sulfamoylbenzophenon-Derivate, Verfahren zu ihrer Herstellung, ihre Verwendung sowie pharmazeutische Präparate auf Basis dieser Verbindungen

Die Erfindung betrifft Verbindungen der Formel V

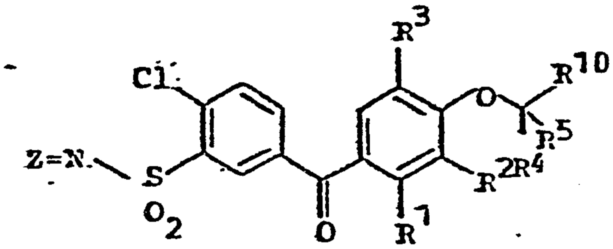

(V) ,

in welcher

$R^1$, $R^2$ und $R^3$ gleich oder verschieden sind und für Wasserstoff, Halogen, Alkyl mit 1 bis 4 C-Atomen oder Alkoxy mit 1 bis 4 C-Atomen stehen, wobei aber $R^1$ und $R^2$ nicht gleichzeitig Wasserstoff bedeuten können und wobei $R^1$ und $R^2$ auch gemeinsam eine Brücke aus zwei bis 5 Methylengruppen oder Methylendioxy bedeuten können,

$R^{10}$ Alkyl mit 1 bis 4 C-Atomen, eine Phenol-Schutzgruppe, Wasserstoff oder einen Rest der Formel VI bedeutet,

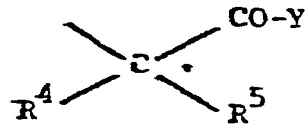

(VI) ,

in der $R^4$ und $R^5$ gleich oder verschieden sind und für Wasserstoff, oder Alkyl mit 1 bis 4 C-Atomen stehen oder Bestandteil eines 3- bis 7-gliedrigen gesättigten carbocyclischen Ringes sind und Y für $OR^6$, worin $R^6$ Wasserstoff, Alkyl mit 1 bis 4 C-Atomen, die gegebenenfalls mit Hydroxy oder Alkoxy mit 1 bis 3 C-Atomen substituiert sein können, oder Aralkyl mit 7 - 9 C-Atomen bedeutet,

oder für eine gegen Lewissäure stabile Schutzgruppe der Carboxylfunktion steht, und

Z für zwei Wasserstoffatome oder eine Schutzgruppe der Formel III,

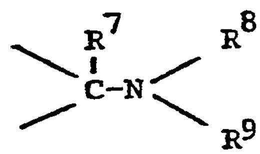

(III),

in der $R^7$ Wasserstoff oder Alkyl mit 1 bis 4 C-Atomen und $R^8$ und $R^9$ Alkyl mit 1 bis 4 C-Atomen bedeuten, steht,

sowie deren physiologisch verträgliche Salze.

In der Literatur sind bereits mehrere in 4-Stellung acylierte Phenoxyessigsäurederivate mit salidiuretischer und/oder urikosurischer Wirkung beschrieben (vgl. G.M. Shutske et. al., J. Med. Chem. 25, 36 - 44 (1982) und dort zit. Lit.). Alle diese Verbindungen besitzen einen lipophilen Aroylteil und wirken an Ratten kaum oder nur in relativ hohen Dosierungen salidiuretisch. Es war daher sehr überraschend, daß erfindungsgemäße Verbindungen der Formel I - die sowohl Phenoxyessigsäure-Derivate als auch Sulfonamide sind - sowohl eine urikosurische als auch eine salidiuretische Wirkung ausüben, und dabei hinsichtlich Aktivität und/oder Wirkdauer die oben erwähnten Verbindungen übertreffen, wie dies durch Versuche an Ratten belegt werden kann.
Weiterhin sind aus dem deutschen Patent 1 129 478 Benzophenonsulfonamid-Derivate mit diuretischen und saluretischen Eigenschaften bekannt, die aber keine urikosurische oder hypourikämischen Effekte aufweisen.

Bevorzugt sind solche Verbindungen der Formel V in welcher

Z zwei Wasserstoffatome, und $R^1$ - $R^6$ die oben genannte Bedeutung haben, insbesondere solche Verbindungen, in denen Z für 2 Wasserstoffatome,

$R^1$, $R^2$ und $R^3$ für Wasserstoff, Halogen und/oder Methyl,

$R^{10}$ für 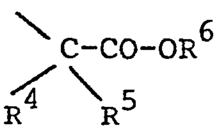 mit

$R^4$ und $R^5$ für Wasserstoff und/oder Methyl, und $R^6$ für Wasserstoff, Alkyl mit 1 bis 4 C-Atomen oder Benzyl steht;

wobei die Verbindungen bevorzugt sind, bei denen

Z zwei Wasserstoffatome,

$R^1$, $R^2$ und $R^3$ Chlor, Methyl und/oder Wasserstoff

$R^{10}$ 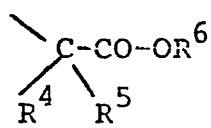 mit

$R^4$ und $R^5$ Wasserstoff und

$R^6$ Wasserstoff oder Alkyl mit 1 bis 4 C-Atomen bedeutet.

Von allen Verbindungen der Erfindung kommen als ganz besondere bevorzugt solche in Betracht, bei denen

Z zwei Wasserstoffatome

$R^{10}$ 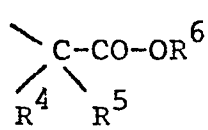,

$R^1$ und $R^2$ Chlor oder Methyl und

$R^3$ bis $R^6$ Wasserstoff

bedeuten.

Die Erfindung betrifft weiterhin ein Verfahren zur Herstellung von Verbindungen der Formel V, das dadurch gekennzeichnet ist, daß man

ein Carbonsäurederivat der Formel II,

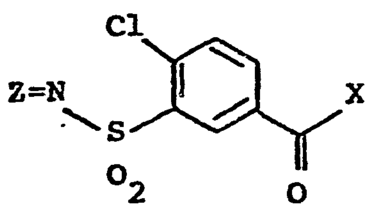

(II),

in der Z für zwei Wasserstoffatome oder für eine Schutzgruppe der Formel III

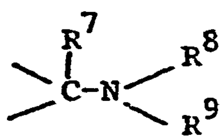

(III),

in welcher $R^7$ Wasserstoff oder Alkyl mit 1 bis 4 C-Atomen und $R^8$ und $R^9$ Alkyl mit 1 bis 4 C-Atomen bedeuten, steht, und in welcher X für eine Leaving-group, insbesondere aber für Chlor steht, mit einem Phenolderivat der allgemeinen Formel IV

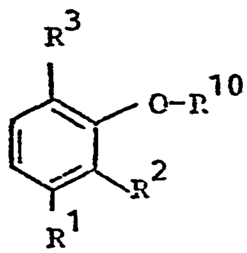

(IV),

in welcher

$R^1$, $R^2$ und $R^3$ die oben genannte Bedeutung haben und

$R^{10}$ a) Alkyl mit 1 bis 4 C-Atomen, eine andere geeignete Phenol-Schutzgruppe oder Wasserstoff bedeutet

oder

b) für einen Rest der Formel VI steht,

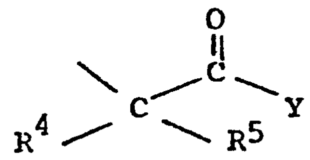

(VI),

in der $R^4$ und $R^5$ die obengenannte Bedeutung haben und Y für $OR^6$, wobei $R^6$ wie oben definiert ist, oder eine gegen Lewissäuren stabile Schutzgruppe der Carboxylfunktion steht,

in an sich bekannter Weise durch Friedel-Crafts-Acylierung zu Verbindungen der allgemeinen Formel V

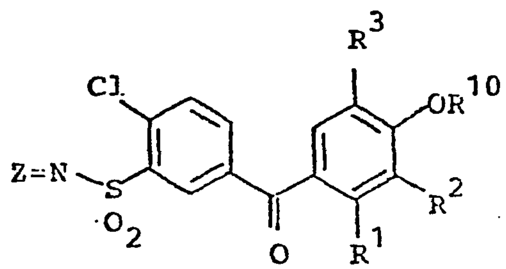

(V),

in welcher $R^1$, $R^2$, $R^3$, $R^{10}$ und Z die obengenannte Bedeutung haben, umsetzt (wobei Verbindungen der Formel V, in der Z 2 Wasserstoffatome und $R^{10}$ ein Rest der Formel VI sind, identisch mit Verbindungen der Formel I sind)

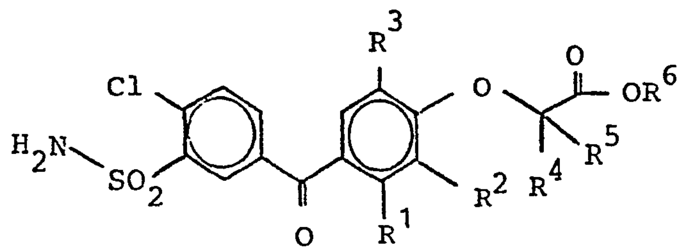

(I)

und diese, falls $R^{10}$ Alkyl mit 1 bis 4 C-Atomen oder eine andere geeignete Phenolschutzgruppe ist, durch Abspaltung des Restes $R^{10}$ in die Phenole der Formel V,

in welcher $R^{10}$ Wasserstoff bedeutet und $R^1$ bis $R^3$ und Z die obengenannte Bedeutung besitzen, überführt, wobei Verbindungen der Formel V, in der Z die Bedeutung einer Schutzgruppe der Formel III hat, sich auf literaturbekannten Wegen (DOS 26 54 795; C.A. 89, 108 715 (1978)) leicht aus Verbindungen mit ungeschützter Sulfonamidgruppe (Z gleich zwei Wasserstoffatome) erhalten oder in diese überführen lassen, die Phenole der vorstehend definierten Formel V mit einem Essigsäurederivat der Formel VII

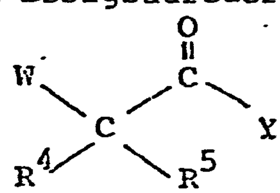 (VII),

in welcher $R^4$, $R^5$ und Y die obengenannte Bedeutung haben und W die Bedeutung einer nucleofugen Gruppe wie beispielsweise Brom, Chlor, Jod, p-Toluolsulfonyl oder Methansulfonyl hat, zu Verbindungen der Formel V, in welcher $R^{10}$ für einen Rest der oben definierten Formel VI steht und die Reste $R^1$ bis $R^5$, Y und Z die obengenannten Bedeutungen aufweisen, alkyliert, diese für den Fall, daß Z die Bedeutung der Schutzgruppe der Formel III hat, leicht durch alkalische oder saure Hydrolyse in die Verbindungen der Formel I überführt und gegebenenfalls diese Verbindungen der Formel I durch saure oder alkalische Verseifung bzw. sauer katalysierte Veresterung oder Umesterungsreaktionen wechselseitig ineinander umwandelt, und gegebenenfalls Verbindungen der Formel I in ihre physiologisch verträglichen Salze überführt.

Die Herstellung der Phenolderivate der Formel V ($R^{10}$= Wasserstoff) geschieht in an sich bekannter Weise dadurch, daß man die Phenolderivate der Formel IV mit Carbonsäurederivaten der Formel II nach Art der Friedel-Crafts-Acylierung (vgl. Houben-Weyl, Bd. VII/2a, S.15-62, Georg Thieme Verlag Stuttgart, 1973) umsetzt, wobei als Friedel-Crafts-Katalysator vorzugsweise Aluminiumchlorid Verwendung findet. Alle für Friedel-Crafts-Acylierungen gängigen Lösungsmittel können Verwendung finden, beson-

BAD ORIGINAL

ders geeignet sind chlorierte Kohlenwasserstoffe wie z.B. Methylenchlorid oder 1,2-Dichlorethan, aber auch Schwefelkohlenstoff kann vorteilhaft sein. Die Umsetzungen werden zwischen -20 und 100°C mit 1-4 Äquivalenten Katalysator vorgenommen, zur Vermeidung von Nebenprodukten arbeitet man vorteilhaft bei Temperaturen unterhalb 40°C und stöchiometrischen Mengen Katalysator.

Zur Überführung der Phenolether der Formel V in die Phenole V ($R^{10}$ = Wasserstoff) eignen sich insbesondere für $R^{10}$ gleich Methyl, alle gängigen Spaltungsreagenzien für Aryl-alkylether, besonders bevorzugt sind Bortribromid und Aluminiumchlorid, wobei als Reaktionsmedien insbesondere chlorierte Kohlenwasserstoffe wie Methylenchlorid und 1,2-Dichlorethan bei Temperaturen zwischen -20 und +80°C, vorzugsweise jedoch zwischen +20 und +50°C, verwendet werden. Aber auch in Schmelzen von wasserfreien Pyridiniumhalogeniden, insbesondere Pyridinhydrochlorid, geht die Spaltung bei Temperaturen zwischen 120 und 200°C, insbesondere zwischen 160 und 190°C, glatt vonstatten.

Zur Vermeidung von Nebenreaktionen werden in die nachfolgende Alkylierungsreaktion vorzugsweise solche Phenole V ($R^{10}$ = Wasserstoff) eingesetzt, deren Sulfonamidgruppe gegen Alkylierung geschützt ist, d.h. in denen Z die Bedeutung von III hat. Es gelingt jedoch auch Phenole der Formel V ($R^{10}$ = Wasserstoff) mit freier Sulfamoylgruppe ziemlich selektiv an der OH-Gruppe zu alkylieren, indem man mit geringem Überschuß an Alkylierungsmitteln der Formel VII und einer schwachen Hilfsbase, vorzugsweise Natriumhydrogencarbonat oder Kaliumcarbonat in einem polaren aprotischen Solvens wie insbesondere Aceton, Methylethylketon, Dimethylformamid, Dimethylacetamid oder N-Methylpyrrolidon vorzugsweise bei Temperaturen zwischen 0 und 50°C umsetzt. Für die Fälle, in denen die resultierenden Verbindungen der Formel V ($R^{10}$ = Gruppe der Formel VI) noch in die Produkte I überführt werden

BAD ORIGINAL

müssen, läßt sich z.B. die Hydrolyse von Estern ($R^6$ in der Bedeutung von Alkyl) zu den freien Phenoxyessigsäuren ($R^6$ gleich H) gleichzeitig mit einer gegebenenfalls erforderlichen Abspaltung der Sulfonamid-Schutzgruppe (Z in der Bedeutung von Formel III) sowohl mit Hilfe einer Base als auch einer Säure in Gegenwart von Wasser vornehmen. Erfolgt die Abspaltung unter sauren Bedingungen, so findet vorzugsweise eine starke Mineralsäure wie Salzsäure oder Schwefelsäure Verwendung. Wird dagegen alkalisch verseift, so werden vorteilhaft starke anorganische Basen wie Alkali- oder Erdalkalibasen wie etwa Lithium-, Natrium- oder Bariumhydroxid verwendet, aber auch starke organische Basen wie quartäre Ammoniumhydroxide, z.B. Tetraethylammoniumhydroxid, können benutzt werden. Als Lösungsmittel kann praktisch jedes, den Reaktionspartnern gegenüber inerte Lösungsmittel, wie z.B. Alkohole, vorzugsweise Methanol, oder, falls sauer hydrolysiert wird, Alkansäuren wie z.B. Essigsäure Verwendung finden. Pro verseifbare Gruppe muß wenigstens ein Äquivalent Wasser dem Reaktionsgemisch zugesetzt werden, meist wird jedoch ein größerer Überschuß verwendet, oder Wasser allein dient als Lösemittel, was insbesondere für alkalische Verseifungen vorteilhaft ist. Die Reaktionstemperatur kann zwischen 0 und 120°C liegen, vorteilhaft wird bei sauren Spaltungen bei der Rückflußtemperatur des Lösungsmittels gearbeitet. Die Isolierung der Reaktionsprodukte geschieht am zweckmäßigsten dadurch, daß man in der Siedehitze ein Nichtlösemittel, wie etwa Wasser, dem Reaktionsgemisch beifügt; das Produkt fällt dann in der Regel beim Abkühlen kristallin an. Bei alkalischer Verseifung arbeitet man im allgemeinen bei Temperaturen zwischen 0 und 50°C, vorzugsweise bei Raumtemperatur, und fällt nach Neutralisation überschüssiger Base durch Zusatz weiterer Säure und ggfs. Kochsalz der freien Säuren der Formel I, $R^6$ = Wasserstoff) aus, filtriert oder extrahiert mit einem geeigneten Lösungsmittel vorzugsweise Essigsäureethylester, und kristallisiert ggfs. um.

In dem folgenden Text bedeutet "Raumtemperatur" 20 - 25°C.

Die physiologisch verträglichen Salze dieser Erfindung umfassen die Salze der Alkali- und Erdalkalimetalle und von nichttoxischen organischen Basen, z.B. von Ethanolamin, Diethanolamin, Trishydroxymethylmethylamin, N-Methylglucamin oder L-Arginin.

Erfindungsgemäß können außer den in den Ausführungsbeispielen beschriebenen Verbindungen auch die in der folgenden Tabellen zusammengestellten Verbindungen der allgemeinen Formel I erhalten werden.

Tabelle 1:

| $R^1$ | $R^2$ | $R^3$ | $R^4$ | $R^5$ | $R^6$ |
|---|---|---|---|---|---|
| $-(CH_2)_4-$ | | H | H | H | H |
| $-(CH_2)_3-$ | | H | H | H | H |
| $-(CH_2)_2-$ | | H | H | H | H |
| $-(CH)_4-$ | | H | H | H | H |
| Cl | | H | H | H | H |
| Cl | H | Cl | H | H | H |
| Cl | Cl | H | $-(CH_2)_2-$ | | H |
| Cl | Cl | H | $-(CH_2)_3-$ | | H |
| Cl | Cl | H | $-(CH_2)_4-$ | | H |
| Cl | $OCH_3$ | Cl | H | H | H |
| $OCH_3$ | Cl | Cl | H | H | H |
| $OCH_3$ | Cl | H | H | H | H |
| OH | Cl | H | H | H | H |
| Cl | Cl | $CH_3$ | H | H | H |
| Cl | Cl | Cl | H | H | H |
| Cl | Cl | $OCH_3$ | H | H | H |
| $CH_3$ | $CH_3$ | Cl | H | H | H |
| Cl | Cl | H | H | H | $CH_2-CH_2-OH$ |
| Cl | Cl | H | H | H | $CH_2-CH_2-O-CH_3$ |

Die erfindungsgemäßen Verbindungen der Formel I sowie deren physiologisch verträglichen Salze sind Diuretika und Saluretika mit zusätzlicher urikosurischer Komponente. Sie können als Pharmazeutika in der Human- und Veterinärmedizin eingesetzt werden. Dazu werden sie in Dosierungen von 1 - 50 mg/kg Körpergewicht und Tag oral, parenteral oder intravenös verabreicht. Für sich allein oder in Kombination mit anderen blutdrucksenkenden, Gefäßerweiternden oder diuretisch wirksamen Substanzen eignen sie sich sowohl zur Behandlung der Hypertonie als auch zur Behandlung von kardial, renal oder hepatisch bedingten Ödemen und anderen auf Störung des Elektrolythaushaltes zurückzuführenden Erscheinungen. Dabei liegt die besondere Bedeutung der obigen Verbindungen in ihrer doppelten Wirksamkeit als Diuretika und Uricosurika. Es ist bekannt, daß während einer diuretischen Behandlung mit bekannten Diuretika in vielen Fällen die Harnsäurekonzentration im Blut eines Patienten steigt. Ein erhöhter Harnsäurespiegel ist ein ernstes Problem bei Gichtpatienten. Darüberhinaus wird ein erhöhter Harnsäurespiegel mehr und mehr als Risikofaktor bei Herzkrankheiten angesehen. Daher kann die diuretische Wirkung mit gleichzeitiger Ausscheidung von Harnsäure als ein Hauptvorteil der erfindungsgemäßen Verbindungen angesehen werden.
Die Verbindungen können allein oder in Kombination mit anderen salidiuretisch wirksamen Substanzen auch anderer Wirkungsart angewendet werden. Insbesondere sind zu nennen: Spironolacton, Triamteren, Amilorid und andere $K^+$-retinierende Verbindungen. Aber auch andere rein blutdrucksenkende Verbindungen kommen als mögliche Kombinationspartner in Frage, z.B. Hydralazin, Clonidin, Reserpin und insbesondere auch beta-blockierende Substanzen wie etwa Metoprolol oder Penbutolol.

Wirksame Mengen der erfindungsgemäßen Verbindungen können einem Patienten auf verschiedene Weise verabreicht

werden, z.B. oral in Form von Kapseln oder Tabletten parenteral in Form von sterilen Lösungen oder Suspensionen und in einigen Fällen auch intravenös als sterile Lösungen.

Die freien Säuren, die selbst wirksam sind, können formuliert werden und aus Gründen der Stabilität, beserer Kristallisierbarkeit, besserer Löslichkeit etc. auch in Form ihrer pharmazeutisch verträglichen Salze verabreicht werden.

Für eine orale Verabreichung können die wirksamen Verbindungen der Erfindung mit einem Verdünnungsmittel oder eßbaren Träger vermischt, in Gelatinekapseln eingeschlossen oder zu Tabletten verpreßt werden. Für eine orale therapeutische Verabreichung können die wirksamen Verbindungen in Träger eingearbeitet und in Form von Tabletten, Pastillen, Kapseln, Elixieren, Suspensionen, Sirups, Waffeln, Kaugummi usw. verwendet werden. Diese Präparate sollen mindestens 0,5 % an wirksamer Substanz enthalten, in Abhängigkeit der besonderen Form kann der Gehalt jedoch zwischen 4 und 70 % des Gewichts der Einheit schwanken. Die Menge an aktiver Verbindung in solchen Präparaten ist so bemessen, daß eine geeignete Dosierung erreicht werden kann. Bevorzugte Mischungen und Präparate enthalten pro oraler Einheitsdosis zwischen 10 und 300 Milligramm der wirksamen Verbindung.

Die Tabletten, Pillen, Kapseln, Pastillen usw. können außerdem die folgenden Bestandteile enthalten: Bindemittel wie mikrokristalline Cellulose, Tragantgummi oder Gelatine; Träger wie Stärke oder Laktose, Zerfallmittel wie Alginsäure, Maisstärke, usw., Gleitmittel wie Magnesiumstearat oder kolloidales Siliziumdioxyd, Süßstoff wie Rohrzucker oder Saccharin, oder

ein Aromastoff wie Pfefferminz, Methylsalicylat oder Orangenaroma.
Im Fall einer Einheitsdosis in Kapselform können diese, neben den genannten Substanzen, auch einen flüssigen Träger enthalten, z.B. ein Öl. Tabletten oder Dragees können z.B. auch mit Zucker, Schellack oder anderen darmlöslichen Überzügen versehen sein. Ein Sirup kann neben der wirksamen Verbindung noch Rohrzucker als Süßstoff, bestimmte Konservierungsmittel, Farbstoffe und Geschmackstoffe enthalten. Die zur Herstellung der Mischungen verwendeten Materialien sollten pharmazeutisch rein und in den zugegebenen Mengen ungiftig sein.

Für eine parenteral therapeutische Verabreichung können die wirksamen Verbindungen gemäß der Erfindung in eine Lösung oder Suspension eingearbeitet werden. Solche Präparate sollen mindestens 0,1 % der aktiven Verbindung enthalten, jedoch kann der Gehalt zwischen 0,5 und 30 Gew.-% schwanken. Die Präparate haben einen solchen Gehalt an wirksamer Verbindung, daß eine geeignete Einheitsdosis erhalten werden kann. Die Mischungen und Präparate gemäß der Erfindung enthalten vorzugsweise zwischen 10 und 500 mg aktive Substanz pro parenteraler Dosiereinheit.

Die Lösungen und Suspensionen können die folgende Komponenten enthalten: ein steriles Verdünnungsmittel, wie Wasser zur Injektion, Salzlösung, nichtflüchtige Öle, Polyethylenglykol, Glycerin, Propylenglykol oder andere synthetische Lösungsmittel, antibakterielle Mittel, wie Benzylalkohol; Antioxydantien wie Ascorbinsäure oder Natriumbisulfit; Chelierungsmittel wie Ethylendiamintetraessigsäure; Puffer, wie Acetate, Zitrate oder Phosphate, und Mittel zur Einstellung der Toxizität wie Kochsalz oder Dextrose. Die parenteralen Präparate können in Ampullen, Einwegspritzen oder Mehrfachdosis-Fläschchen aus Glas oder Plastik abgefüllt werden.

Die folgenden Beispiele veranschaulichen die Erfindung.

Beispiel 1:

2,3-Dichlor-4-(4-chlor-3-sulfamoylbenzoyl)-phenoxyessigsäure

a) 4-Methoxy-3'-sulfamoyl-2,3,4'-trichlorbenzophenon

In eine Suspension von 610 g (4,6 Mol) Aluminiumchlorid gibt man portionsweise unter Rühren bei -10°C 635 g (2,5 Mol) 4-Chlor-3-sulfamoyl-benzoesäurechlorid. Man rührt ca. 30 Min. bei -10 bis -5°C und tropft dann eine Lösung von 354 g (2,0 Mol) 2,3-Dichloranisol in 0,6 l Methylenchlorid zu.

Man rührt die erhaltene Mischung bei Raumtemperatur bis die HCl-Entwicklung beendet ist (2 - 5 Tage), gießt auf eine Mischung aus ca. 6 kg Eis und 1,5 l konzentrierter Salzsäure, nutscht den ausgefallenen Feststoff ab, wäscht ihn gut mit Wasser, Natriumhydrogencarbonat-Lösung und wieder Wasser, trocknet ihn und kristallisiert aus Aceton, Ethanol oder Dimethylformamid-Wasser-Mischung um. Farblose Kristalle vom Schm. 219 - 221°C.

b) 4-Hydroxy-3'-sulfamoyl-2,3,4'-trichlorbenzophenon

In eine gerührte Suspension von 21 g (0,053 Mol) 4-Methoxy-3'-sulfamoyl-2,3,4'-trichlorbenzophenon in ca. 400 ml Toluol tropft man bei -20°C 25 ml (0,26 Mol) Bortribromid. Nach 5-tägigem Rühren bei Raumtemperatur gießt man die rotbraune Mischung unter kräftigem Rühren auf ca. 0,7 l eiskalte gesättigte Natriumhydrogencarbonat-Lösung, trennt den beigefarbenen Feststoff durch Abnutschen ab, wäscht mit verdünnter Salzsäure und Wasser und trocknet im Vakuumexsikkator über Phosphorpentoxid. Cremefarbenes Pulver vom Schmp. 214 - 218°C (Zers.)

Ein nahezu gleichwertiges Produkt erhält man auch durch ca. 3-stündiges Erhitzen einer Mischung des gleichen Ausgangsmaterials mit 2,5 Gewichtsteilen wasserfreiem Pyridinhydrochlorid auf 180°C unter Stickstoff und anschließendes Eintragen der noch flüssigen Schmelze in eine Eis-Salzsäure-Mischung, Waschen und Trocknen des anfallenden beigefarbenen Feststoffes.

c) 2,3-Dichlor-4-(4-Chlor-3-sulfamoylbenzoyl)-phenoxy-essigsäuremethylester

Eine Mischung aus 58 g (0,152 Mol) 4-Hydroxy-3'-sulfamoyl-2,3,4'-trichlorbenzophenon, 21 g (0,152 Mol) gemahlenem Kaliumcarbonat und 2,2 g (0,015 Mol) Natriumiodid wird in ca. 1,2 l entgastem Methylethylketon gelöst bzw. suspendiert. Man läßt ganz langsam bei Raumtemperatur 29 g (0,195 Mol) Bromessigsäuremethylester zutropfen, läßt bis zur vollständigen Umsetzung (dünnschichtchromatographische Kontrolle, 2 - 6 Tage) nachrühren, filtriert, wäscht den Filterkuchen gut mit warmem Aceton, engt das Filtrat am Rotationsverdampfer zur Trockne ein, digeriert den Rückstand bis zur Kristallisation mit Wasser, trennt den Feststoff ab, kocht ihn mit wenig Methanol auf, nutscht nach dem Abkühlen ab und trocknet in gutem Vakuum. Weißes bis cremefarbenes Pulver vom Schmp. 191 - 194°C. Nach Umkristallisation aus Methanol-Aceton: Farblose Kristalle vom Schmp. 203 - 206°C.

d) 2,3-Dichlor-4-(4-chlor-3-sulfamoylbenzoyl)-phenoxy-essigsäure

31,6 g (70 mMol) 2,3-Dichlor-4-(4-chlor-3-sulfamoylbenzoyl)-phenoxyessigsäuremethylester (1c) werden in ca. 700 ml Methanol suspendiert. Bei 0°C werden innerhalb einer Stunde 1 l 0,15 N wäßrige Lithiumhydroxid-Lösung unter Rühren zugetropft. Man läßt über Nacht bei einer Temperatur zwischen 0 und 10°C rühren, versetzt die klare Lösung dann mit 1,5 kg Eis und 0,5 l gesättigter Kochsalzlösung, stellt durch tropfenweise Zugabe von 2 N Salzsäure (ca. 70 ml) auf pH 3, zentrifugiert den voluminösen hellen Niederschlag ab, wäscht das Zentrifugat einmal mit Wasser und kristallisiert aus einer Essigsäure-Wasser-Mischung unter Zusatz von Aktivkohle um und trocknet bei 80°C und 150 Torr über Phosphorpentoxid. Farblose Blättchen vom Schmp. 184 - 6°C.

Beispiel 2:

5-Chlor-2-methyl-4-(4-chlor-3-sulfamoylbenzoyl)-phenoxyessigsäure

Die Verbindung wird analog der in Beispiel 1 beschriebenen Reaktionsfolge, jedoch mit 5-Chlor-2-methylanisol als Ausgangsmaterial hergestellt. Die Zwischenprodukte und das Endprodukt weisen folgende Schmelzpunkte auf:

a) 2,4'-Dichlor-4-methoxy-5-methyl-3'-sulfamoylbenzophenon, Schmp. 172 - 173°C

b) 2,4'-Dichlor-4-hydroxy-5-methyl-3'-sulfamoylbenzophenon, Schmp. 230 - 234°C

c) 5-Chlor-2-methyl-4-(4-chlor-3-sulfamoylbenzoyl)-phenoxyessigsäuremethylester, Schmp. 165 - 169°C

d) 5-Chlor-2-methyl-4-(4-chlor-3-sulfamoylbenzoyl)-phenoxyessigsäure, Schmp. 224 - 246°C

Beispiel 3:

3-Chlor-2-methyl-4-(4-chlor-3-sulfamoylbenzoyl)-phenoxyessigsäure

Die Verbindung wird analog der in Beispiel 1 beschriebenen Reaktionsfolge, jedoch mit 3-Chlor-2-methylanisol als Ausgangsmaterial hergestellt. Die Zwischenprodukte und das Endprodukt weisen folgende Schmelzpunkte auf:

a) 2,4'-Dichlor-4-methoxy-3-methyl-3'-sulfamoylbenzophenon, Schmp. 222 - 224°C

b) 2,4'-Dichlor-4-hydroxy-3-methyl-3'-sulfamoylbenzophenon, Schp. 220 - 224°C

c) 3-Chlor-2-methyl-4-(4-chlor-3-sulfamoylbenzoyl)-phenoxyessigsäuremethylester, Schmp. 192 - 196°C

d) 3-Chlor-2-methyl-4-(4-chlor-3-sulfamoylbenzoyl)-phenoxyessigsäure, Schmp. 182 - 4°C

Beispiel 4:

Die Verbindung wird analog der in Beispiel 1 beschriebenen Reaktionfolge, jedoch mit 2-Chlor-3-methylanisol als Ausgangsmaterial hergestellt. Die Zwischenprodukte und das Endprodukt weisen folgende Schmelzpunkte auf:

a) 3,4'-Dichlor-4-methoxy-2-methyl-3'-sulfamoylbenzophenon, Schmp. 186 - 188°C

b) 3,4'-Dichlor-4-hydroxy-2-methyl-3'-sulfamoylbenzophenon, Schmp. 194 - 197°C

c) 2-Chlor-3-methyl-4-(4-chlor-3-sulfamoylbenzoyl)-phenoxyessigsäuremethylester, Schmp. 152 - 154°C

d) 2-Chlor-3-methyl-4-(4-chlor-3-sulfamoylbenzoyl)-phenoxyessigsäure, Schmp. 178 - 180°C

Beispiel 5 :

2,3-Dichlor-4-(4-chlor-3-sulfamoylbenzoyl)-phenoxyessigsäureethylester

a) Die Verbindung wird analog der in Beispiel 1 unter 1c beschrieben Vorgehensweise hergestellt, jedoch wird Chloressigsäureethylester an Stelle von Bromessigsäuremethylester verwendet und das Produkt durch Säulenchromatographie an Kieselgel mit $CH_2Cl_2$-Essigester als Elutionsmittel gereinigt und aus Ethanol-Wasser umkristallisiert. Farblose Kristalle vom Schmp. 146 - 148°C.

b) 4,4 g (10 mmol) 2,3-Dichlor-4-(4-chlor-3-sulfamoylbenzoyl)-phenoxyessigsäure (Beispiel 1) werden mit 150 ml Ethanol, 0,2 g stark saurem Ionenaustauscherharz (Dowex 50 W) und 50 ml Toluol versetzt und bis zum Ende der Wasserabscheidung am Wasserabscheider unter Rückfluß gerührt (ca. 4 h). Vom Ionenaustauscher wird durch Filtration abgetrennt, das Filtrat im Vakuum eingeengt und der Rückstand aus einer Ethanol /Aceton/Wasser-Mischung umkristallisiert. Farblose Kristalle vom Schmp. 145 - 148°C.

Beispiel 6 :

2,3-Dichlor-4-(4-chlor-3-sulfamoylbenzoyl)-phenoxyessigsäure-tert-butylester

Die Verbindung wird analog der in Beispiel 1 unter 1c beschriebenen Arbeitsweise hergestellt, wobei man jedoch Bromessigsäure-tert-butylester an Stelle vom Bromessigsäuremethylester verwendet und das Produkt nach säulenchromatographischer Reinigung an Kieselgel mit $CH_2Cl_2$-Essigester (20 1 bis 3:1) als Eluens aus Isopropanol-Wasser umkristallisiert und getrocknet wird. Blaßgelbe Kristalle vom Schmp. 145 - 147°C.

Beispiel 7 :

2,3-Dichlor-4-(4-chlor-3-sulfamoylbenzoyl)-phenoxyessigsäuremethylester

Die Verbindung ist identisch mit dem in Beispiel 1 unter 1c beschriebenen Zwischenprodukt und kann wie dort beschrieben hergestellt oder auf folgendem Weg erhalten werden:
4,4 g (10 mmol) 2,3-Dichlor-4-(4-chlor-3-sulfamoylbenzoyl)-phenoxyessigsäure (nach Beispiel 1 oder Beispiel 16 erhalten) werden in 200 ml wasserfreiem Methanol gelöst und nach Zusatz von 1 ml konzentrierter Schwefelsäure ca. einen Tag bei Raumtemperatur stehengelassen. Man saugt den ausgefallenen Feststoff ab und trocknet ihn im Vakuum. Farblose Kristalle vom Schmp. 208 - 210°C.

Beispiel 8:

2,3-Dimethyl-4-(4-chlor-3-sulfamoylbenzoyl)-phenoxy-essigsäure

40 g (0,3 Mol) Aluminiumchlorid werden in 300 ml Methylenchlorid suspendiert und bei Eisbadtemperatur unter Feuchtigkeitsausschluß 25,4 g (0,1 Mol) 4-Chlor-3-sulfamoylbenzoylchlorid eingerührt. Man läßt 45 Minuten nachrühren und gibt dann bei 0°C unter Rühren 18 g (0,1 Mol) 2,3-Dimethylphenoxyessigsäure portionsweise zu. Man läßt bei Raumtemperatur weiterrühren wobei die Mischung allmählich viskos wird. Nach mehrtägigem Stehen wird mit Eis-Wasser-Salzsäure-Mischung zersetzt, die wäßr. Phase mit Essigsäureethylester mehrmals ausgeschüttelt, die organischen Phasen getrocknet, am Rotationsverdampfer eingeengt und der harzige Rückstand zunächst aus Isopropanol und dann aus wäßriger Essigsäure, jeweils unter Zusatz von Aktivkohle, umkristallisiert. Farblose Kristalle vom Schmp. 186-190°C.

Beispiel 9:

2,3-Dimethyl-4-(4-Chlor-3-sulfamoylbenzoyl)-phenoxy-essigsäuremethylester

4,0 g (10 mMol) 2,3-Dimethyl-4-(4-chlor-3-sulfamoyl-benzoyl)-phenoxyessigsäure (Beispiel 9) werden in der gerade ausreichenden Menge siedendem Methanol gelöst und 15 Min. weitererhitzt. Die nach langsamen Abkühlen und Anreiben erhaltenen farblosen Kristalle werden gesammelt und im Vakuum vom Lösungsmittel befreit, Schmp. 157 - 162°C.

Beispiel 10:

3-Methyl-4-(4-chlor-3-sulfamoylbenzoyl)phenoxyessigsäure

Die Verbindung wird analog Beispiel 9, jedoch mit 3-Methylphenoxyessigsäure als Ausgangsmaterial, hergestellt. Farblose Kristalle vom Schmp. 208 - 211°C

Beispiele 11:

2-Methyl-4-(4-chlor-3-sulfamoylbenzoyl)-phenoxyessigsäure

Diese Verbindung wird analog dem in Beispiel 9 beschriebenen Weg, jedoch mit 2-Methylphenoxyessigsäure als Ausgangsmaterial hergestellt. Farblose Kristalle vom Schmp. 198 - 200°C

Beispiele 12:

2,6-Dimethyl-4-(4-chlor-3-sulfamoylbenzoyl)-phenoxyessigsäure

Die Verbindung wird analog Beispiel 9, jedoch mit 2,6-Dimethylphenoxyessigsäure als Ausgangsmaterial, hergestellt. Farblose Kristalle vom Schmp. 237 - 240°C.

Beispiel 13:

2-Chlor-4-(4-chlor-3-sulfamoylbenzoyl)-phenoxyessigsäure

Die Verbindung wird analog Beispiel 9 hergestellt, wobei jedoch 2-Chlorphenoxyessigsäure an Stelle von 2,3-Dimethylphenoxyessigsäure als Ausgangsmaterial verwendet wird und die Komponenten in 1,2-Dichlorethan ca. 3 Tage bei ca. 50°C gerührt werden. Das Rohprodukt wird durch mehrere aufeinanderfolgende Umkristallisationen aus Wasser-Aceton und Isopropanol-Toluol gereinigt. Weiße Kristalle vom Schmp. 163 - 167°C.

Beispiel 14 :

2,3-Dichlor-4-(4-chlor-3-sulfamoylbenzoyl)-phenoxyessigsäure

a) 4-Methoxy-3'-dimethylaminomethylenaminosulfonyl-2,3,4'-trichlorbenzophenon

19,7 g (50 mMol) 4-Methoxy-3'-sulfamoyl-2,3,4'-trichlorbenzophenon werden in 200 ml Methylenchlorid suspendiert und bei Raumtemperatur unter Feuchtigkeitsausschluß mit 20 ml Dimethylformamiddimethylacetal versetzt. Es tritt unter Rühren rasch Lösung ein, die über Nacht stehengelassen wird. Einengen zur Trockne und Umkristallisation des Rückstandes aus Isopropanol-Dimethylformamid ergibt derbe blaßgelbe Kristalle vom Schmp. 200 - 202°C.

b) 4-Hydroxy-3'-dimethylaminomethylenaminosulfonyl-2,3,4'-trichlorbenzophenon

45 g (0,1 Mol) 4-Methoxy-3'-dimethylaminomethylenaminosulfonyl-2,3,4'-trichlorbenzophenon (Stufe a) werden in 250 ml trockenem 1,2-Dichlorethan suspendiert und unter Stickstoffatmosphäre gerührt. Portionsweise werden 54,2 g (0,4 Mol) Aluminiumchlorid zugesetzt, wobei die Temperatur auf ca. 35°C steigt und die Mischung nahezu homogen wird. Bei 40 - 50°C wird so lange gerührt, bis im Dünnschichtchromatogramm kein Ausgangsmaterial mehr nachweisbar ist (ca. 5 Stdn.). Zur mittlerweile harzigen Mischung wird unter Außenkühlung rasch Eiswasser gerührt und bis zur Abscheidung eines kristallinen Feststoffs nachgerührt. Dieser wird abgesaugt, mit Wasser neutral gewaschen, getrocknet und aus Methanol unter Zusatz von Aktivkohle umkristallisiert. Graugelbe Kristalle vom Schmp. 196 - 198°C.

c) 2,3-Dichlor-4-(4-chlor-3-dimethylaminomethylenaminosulfonylbenzyl)-phenoxyessigsäuremethylester

4,3 g (10 mMol) 4-Hydroxy-3'-dimethylaminomethylenaminosulfonyl-2,3,4'-trichlorbenzophenon (Stufe 6), 1,7 g (12 mMol) fein gemahlenes Kaliumcarbonat, 0,75 g Natriumiodid 20 ml trockenes Dimethylformamid und 1,8 g (13 mMol) Bromessigsäuremethylester werden in dieser Reihenfolge zusammengegeben und unter dünnschichtchromatographischer Kontrolle bis zur vollständigen Umsetzung

(ca. 4 Stdn.) bei Raumtemperatur heftig gerührt. Zur Aufarbeitung gießt man auf eiskalte verdünnte Salzsäure, saugt den hellgelben Feststoff ab und kristallisiert ihn aus Methanol-Essigester. Farblose Kristalle vom Schmp. 130 - 132°C.

d) 2,3-Dichlor-4-(4-chlor-3-sulfamoylbenzoyl)-phenoxyessigsäure

44,5 g (0,09 Mol) 2,3-Dichlor-4-(4-chlor-3-dimethylaminomethylenaminosulfonylbenzoyl)-phenoxyessigsäuremethylester (Stufe c) werden in einer Mischung aus 150 ml Essigsäure, 450 ml Wasser und 45 ml konzentrierter Salzsäure 3 Stunden unter Rückfluß gerührt, wobei eine klare farblose Lösung entsteht. Bei langsamen Abkühlenlassen entsteht ein feinkristalliner Niederschlag, der abgenutscht und gegebenenfalls unter Zusatz von Aktivkohle aus Essigsäure-Wasser umkristallisiert wird. Farblose Kristalle vom Schmp. 186 - 188°C.

Beispiel 15:

2-[2,3-Dichlor-4-(4-chlor-3-sulfamoylbenzoyl)]-phenoxypropionsäure

Die Verbindung wird analog der in Beispiel 15 beschriebenen Reaktionsfolge, jedoch in Stufe c) mit 2-Brom-propionsäureethylester als Alkylierungsmittel (bei 50°C), hergestellt. Das Zwischenprodukt und das Endprodukt weisen folgende Schmelzpunkte auf:

c) 2-[2,3-Dichlor-4-(4-chlor-3-dimethylaminomethylenaminosulfonylbenzoyl)]-phenoxypropionsäure-ethylester, Schmp. 141 - 143°C

d) 2-[2,3-Dichlor-4-(4-chlor-3-sulfamoylbenzoyl)]1-phenoxy-propionsäure, Schmp. ca. 145°C (hält hartnäckig Lösungsmittelreste fest)

Beispiel 16:

2-[2,3-Dichlor-4-(4-chlor-3-sulfamoylbenzoyl)]-phenoxy-2-methylpropionsäure

Die Verbindung wird analog der in Beispiel 15 beschriebenen Reaktionsfolge, jedoch in Stufe c) mit 2-Brom-2-methylpropionsäure-ethylester als Alkylierungsmittel bei einer Reaktionstemperatur von ca. 60°C und ca. 20 Std. Reaktionsdauer, hergestellt. Das Zwischenprodukt (nach säulenchromatographischer Reinigung) und das Endprodukt weisen folgende Schmelzpunkte auf:

c) 2-[2,3-Dichlor-4-(4-chlor-3-dimethylaminomethylenamino-sulfonylbenzoyl)]phenoxy-2-methylpropionsäure-ethylester Schmp. 112 - 114°C

d) 2-[2,3-Dichlor-4-(4-chlor-3-sulfamoylbenzoyl)]-phenoxy-2-methylpropionsäure, Schmp. 219 - 220°C.

Patentansprüche:

1. Verbindung der Formel V

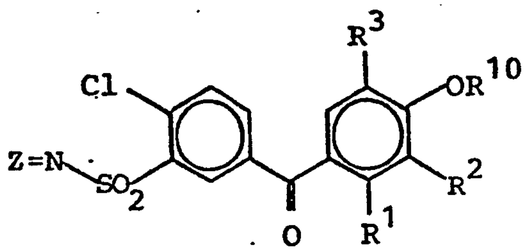

(V),

in welcher

$R^1$, $R^2$ und $R^3$ gleich oder verschieden sind und für
Wasserstoff,
Halogen
Alkyl mit 1 bis 4 C-Atomen oder
Alkoxy mit 1 bis 4 C-Atomen stehen,

wobei aber $R^1$ und $R^2$ nicht gleichzeitig Wasserstoff bedeuten können und wobei

$R^1$ und $R^2$ auch gemeinsam eine Brücke aus 2 bis 5 Methylengruppen oder Methylendioxy

bedeuten können,

$R^{10}$
Alkyl mit 1-4 C-Atomen,
eine Phenolschutzgruppe,
Wasserstoff oder
einen Rest der Formel VI bedeutet

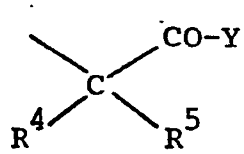

(VI),

in der

$R^4$ und $R^5$ gleich oder verschieden sind und für
Wasserstoff oder
Alkyl mit 1 bis 4 C-Atomen
stehen oder
Bestandteil eines 3- bis 7-gliedrigen gesättigten carbocyclischen Ringes sind,
und

Y für $OR^6$, worin
$R^6$
Wasserstoff,
Alkyl mit 1-4 C-Atomen, das gegebenenfalls mit
Hydroxy oder
Alkoxy mit 1-3 C-Atomen
substituiert sein kann,
oder $R^6$
Aralkyl mit 7-9 C-Atomen
bedeutet, oder

Y für eine gegen Lewissäure stabile Schutzgruppe der Carboxylfunktion steht,
und

Z für
2 Wasserstoffatome oder
eine Schutzgruppe der Formel III

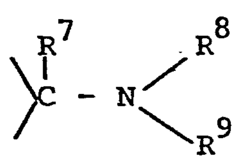

(III)

steht, in der

$R^7$ Wasserstoff oder Alkyl mit 1-4 C-Atomen und

$R^8$ und $R^9$ Alkyl mit 1-4 C-Atomen bedeuten.

2. Verbindung gemäß Anspruch 1 der Formel I

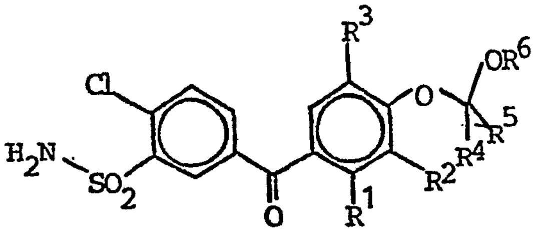

(I),

in der $R^1$, $R^2$, $R^3$, $R^4$, $R^5$ und $R^6$ die in Anspruch 1 definierte Bedeutung haben.

3. Verbindung der Formel V nach Anspruch 1, dadurch gekennzeichnet, daß
Z 2 Wasserstoffatome bedeutet, $R^1$, $R^2$ und $R^3$ für Wasserstoff, Halogen und/oder Methyl stehen, $R^{10}$ für

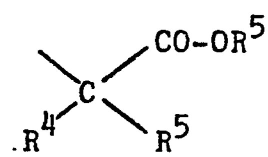

mit $R^4$ und $R^5$ für Wasserstoff und/oder Methyl und $R^6$ für Wasserstoff, Alkyl mit 1-4 C-Atomen oder Benzyl steht.

4. Verbindung der Formel V gemäß Anspruch 1, dadurch gekennzeichnet, daß

| | |
|---|---|
| Z | 2 Wasserstoffatome, |
| $R^1$, $R^2$ und $R^3$ | Chlor, Methyl und/oder Wasserstoff, |
| $R^{10}$ 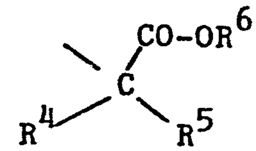 | mit $R^4$ und $R^5$ gleich Wasserstoff und $R^6$ gleich Wasserstoff oder Alkyl mit 1-4 C-Atomen |

bedeutet.

5. Verbindung der Formel V gemäß Anspruch 1, dadurch gekennzeichnet, daß

| | |
|---|---|
| Z | 2 Wasserstoffatome, |
| $R^{10}$ | 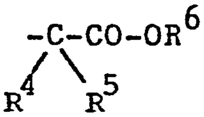, |
| $R^1$ und $R^2$ | Chlor oder Methyl und |
| $R^3$ bis $R^6$ | Wasserstoff |

bedeutet.

6. Verbindung der Formel V gemäß Anspruch 1, dadurch gekennzeichnet, daß Z 2 Wasserstoffatome und $R^1$, $R^2$, $R^3$ die in Anspruch 1 angegebene Bedeutung haben und daß $R^{10}$ Wasserstoff bedeutet.

7. Verfahren zur Herstellung von Verbindungen der Formel V gemäß Anspruch 1, dadurch gekennzeichnet, daß man ein Carbonsäurederivat der Formel II,

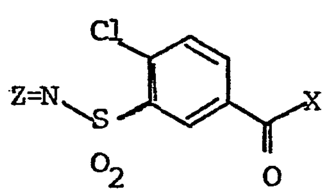

(II) ,

in der Z die in Anspruch 1 definierte Bedeutung hat und X für eine "Leaving-group" steht, mit einem Phenolderivat der allgemeinen Formel IV

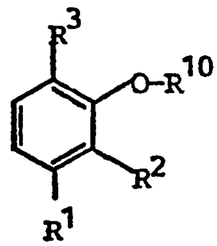

(IV) ,

in welcher

$R^1$, $R^2$ und $R^3$ die in Anspruch 1 definierte Bedeutung haben und

$R^{10}$
a) Alkyl mit 1 bis 4 C-Atomen,
b) eine andere geeignete Phenol-Schutzgruppe oder
c) Wasserstoff bedeutet
oder
d) für einen Rest der Formel VI steht,

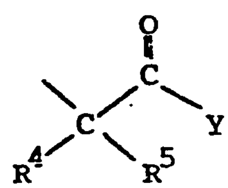

(VI),

in der $R^4$ und $R^5$ die in Anspruch 1 definierte Bedeutung haben und Y für $OR^6$, wobei $R^6$ wie im Anspruch 1 definiert ist, oder eine gegen Lewissäure stabile Schutzgruppe der Carboxylfunktion steht,

in an sich bekannter Weise zu Verbindungen der allgemeinen Formel V

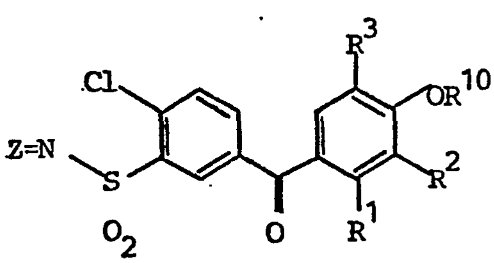

(V) ,

in welcher
$R^1$, $R^2$, $R^3$, $R^{10}$ und Z die oben genannte Bedeutung haben, umsetzt (wobei Verbindungen der Formel V, in der Z 2 Wasserstoffatome und $R^{10}$ einen Rest der Formel VI bedeuten, identisch mit Verbindungen der Formel I sind) und diese, falls $R^{10}$ Alkyl mit 1 bis 4 C-Atomen oder eine andere geeignete Phenolschutzgruppe ist, durch Abspaltung des Restes $R^{10}$ in die Phenole der Formel V, in welcher $R^{10}$ Wasserstoff bedeutet und $R^1$ bis $R^3$ und Z die obengenannte Bedeutung besitzen, überführt, wobei Verbindungen der Formel V, in der Z die Bedeutung einer Schutzgruppe der Formel III hat, sich in an sich bekannter Weise aus Verbindungen mit ungeschützter Sulfonamidgruppe (Z gleich zwei Wasserstoffatome) erhalten oder in diese überführen lassen, die Phenole der vorstehend definierten Formel V mit einem Essigsäurederivat der Formel VII

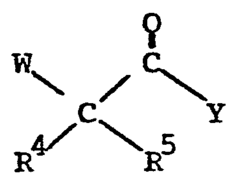

(VII),

in welcher $R^4$, $R^5$ und Y die oben genannte Bedeutung haben und W die Bedeutung einer nucleofugen Gruppe hat, zu Verbindungen der Formel V, in welcher $R^{10}$ für einen Rest der oben definierten Formel VI steht und die Reste $R^1$ bis $R^5$, Y und Z die oben genannten Bedeutungen aufweisen, alkyliert, und diese für den Fall, daß Z die Bedeutung der Schutzgruppe der Formel III hat, durch alkalische oder saure Hydrolyse in die Verbindungen der Formel I überführt, gegebenenfalls diese Verbindungen der Formel I durch saure oder alkalische Verseifung bzw. sauer katalysierte Veresterung oder Umesterungsreaktionen wechselseitig ineinander umwandelt
und
Verbindungen der Formel V gegebenenfalls in ihre physiologisch verträglichen Salze überführt.

8. Verbindungen gemäß einem der Ansprüche 2 bis 5 zur Verwendung als Heilmittel.

9. Mittel enthaltend eine Verbindung gemäß einem der Ansprüche 2 bis 5.

10. Verwendung einer Verbindung gemäß einem der Ansprüche 2 bis 5 als Heilmittel.

Patentansprüche Österreich: HOE 82/F 174

1. Verfahren zur Herstellung von Verbindungen der Formel V

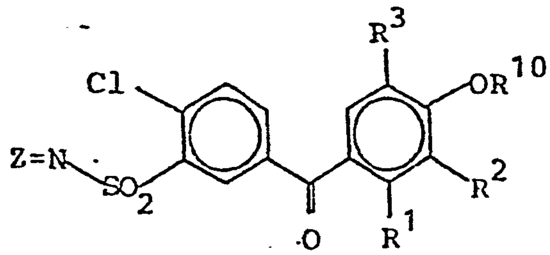

(V),

in welcher

$R^1$, $R^2$ und $R^3$ gleich oder verschieden sind und für Wasserstoff, Halogen Alkyl mit 1 bis 4 C-Atomen oder Alkoxy mit 1 bis 4 C-Atomen stehen,

wobei aber $R^1$ und $R^2$ nicht gleichzeitig Wasserstoff bedeuten können und wobei

$R^1$ und $R^2$ auch gemeinsam eine Brücke aus 2 bis 5 Methylengruppen oder Methylendioxy bedeuten können,

$R^{10}$ Alkyl mit 1-4 C-Atomen, eine Phenolschutzgruppe, Wasserstoff oder einen Rest der Formel VI bedeutet

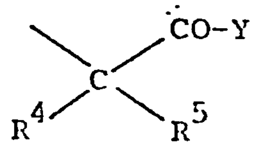

(VI),

in der

$R^4$ und $R^5$ gleich oder verschieden sind und für
Wasserstoff oder
Alkyl mit 1 bis 4 C-Atomen
stehen oder
Bestandteil eines 3- bis 7-gliedrigen gesättigten carbocyclischen Ringes sind,
und

Y für $OR^6$, worin
$R^6$
Wasserstoff,
Alkyl mit 1-4 C-Atomen, das gegebenenfalls mit
Hydroxy oder
Alkoxy mit 1-3 C-Atomen
substituiert sein kann,
oder $R^6$
Aralkyl mit 7-9 C-Atomen
bedeutet, oder

Y für eine gegen Lewissäure stabile Schutzgruppe der Carboxylfunktion steht,
und

Z für
2 Wasserstoffatome oder
eine Schutzgruppe der Formel III

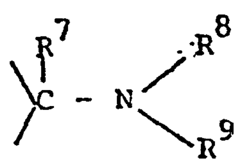

(III)

steht, in der

$R^7$ Wasserstoff oder Alkyl mit 1-4 C-Atomen und

$R^8$ und $R^9$ Alkyl mit 1-4 C-Atomen bedeuten,

dadurch gekennzeichnet, daß man ein Carbonsäurederivat der Formel II,

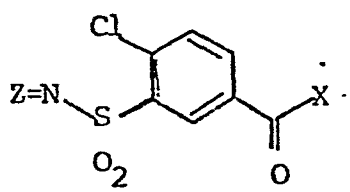

(II) ,

in der Z die oben definierte Bedeutung hat und X für eine "Leaving-group" steht, mit einem Phenolderivat der allgemeinen Formel IV

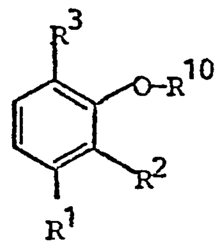

(IV) ,

in welcher $R^1$, $R^2$, $R^3$ und $R^{10}$ die oben definierte Bedeutung haben in an sich bekannter Weise zu Verbindungen der allgemeinen Formel V

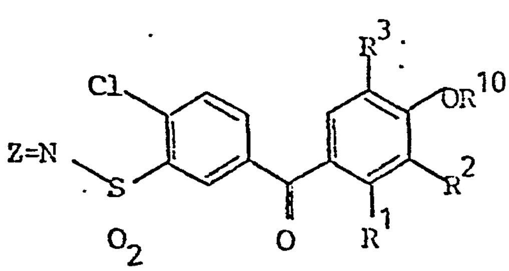

(V) ,

in welcher
$R^1$, $R^2$, $R^3$, $R^{10}$ und Z die oben genannte Bedeutung haben, umsetzt (wobei Verbindungen der Formel V, in der Z 2 Wasserstoffatome und $R^{10}$ einen Rest der Formel VI bedeuten, identisch mit Verbindungen der Formel I sind) und diese, falls $R^{10}$ Alkyl mit 1 bis 4 C-Atomen oder eine andere geeignete Phenolschutzgruppe ist, durch Abspaltung des Restes $R^{10}$ in die Phenole der Formel V, in welcher $R^{10}$ Wasserstoff bedeutet und $R^1$ bis $R^3$ und Z die obengenannte Bedeutung besitzen, überführt, wobei Verbindungen der Formel V, in der Z die Bedeutung einer Schutzgruppe der Formel III hat, sich in an sich bekannter Weise aus Verbindungen mit ungeschützter Sulfonamidgruppe (Z gleich zwei Wasserstoffatome) erhalten oder in diese überführen lassen, die Phenole der vorstehend definierten Formel V mit einem Essigsäurederivat der Formel VII

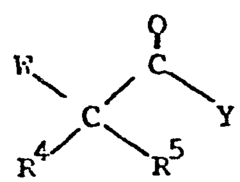

(VII),

in welcher $R^4$, $R^5$ und Y die oben genannte Bedeutung haben und W die Bedeutung einer nucleofugen Gruppe hat, zu Verbindungen der Formel V, in welcher $R^{10}$ für einen Rest der oben definierten Formel VI steht und die Reste $R^1$ bis $R^5$, Y und Z die oben genannten Bedeutungen aufweisen, alkyliert, und diese für den Fall, daß Z die Bedeutung der Schutzgruppe der Formel III hat, durch alkalische oder saure Hydrolyse in die Verbindungen der Formel I überführt, gegebenenfalls diese Verbindungen der Formel I durch saure oder alkalische Verseifung bzw. sauer katalysierte Veresterung oder Umesterungsreaktionen wechselseitig ineinander umwandelt
und
Verbindungen der Formel V gegebenenfalls in ihre physiologisch verträglichen Salze überführt.

2. Verfahren nach Anspruch 1, dadurch gekennzeichnet, daß man eine Verbindung der Formel I

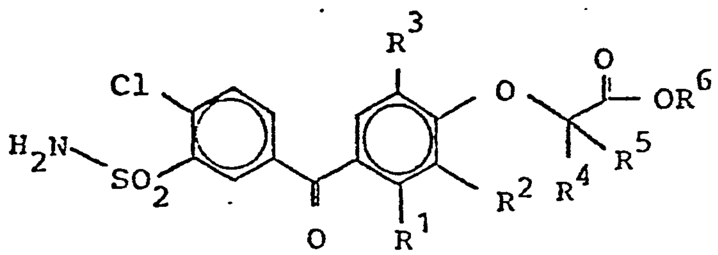

(I)

herstellt,
in der $R^1$, $R^2$, $R^3$, $R^4$, $R^5$ und $R^6$ die in Anspruch 1 definierte Bedeutung haben.

3. Verfahren zur Herstellung einer Verbindung der Formel V nach Anspruch 1, dadurch gekennzeichnet, daß
Z 2 Wasserstoffatome bedeutet, $R^1$, $R^2$ und $R^3$ für Wasserstoff, Halogen und/oder Methyl stehen,
$R^{10}$ für

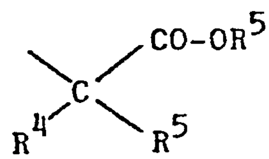

mit $R^4$ und $R^5$ für Wasserstoff und/oder Methyl und $R^6$ für Wasserstoff, Alkyl mit 1-4 C-Atomen oder Benzyl steht.

4. Verfahren zur Herstellung einer Verbindung der Formel V gemäß Anspruch 1, dadurch gekennzeichnet, daß

Z 2 Wasserstoffatome,
$R^1$, $R^2$ und $R^3$ Chlor, Methyl und/oder Wasserstoff,
$R^{10}$

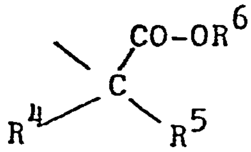

mit $R^4$ und $R^5$ gleich Wasserstoff und $R^6$ gleich Wasserstoff oder Alkyl mit 1-4 C-Atomen bedeutet.

5. Verfahren zur Herstellung einer Verbindung der Formel V gemäß Anspruch 1, dadurch gekennzeichnet, daß

| | |
|---|---|
| Z | 2 Wasserstoffatome, |
| $R^{10}$ | 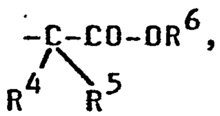 |
| $R^1$ und $R^2$ | Chlor oder Methyl und |
| $R^3$ bis $R^6$ | Wasserstoff |

bedeutet.

6. Verfahren zur Herstellung einer Verbindung der Formel V gemäß Anspruch 1, dadurch gekennzeichnet, daß Z 2 Wasserstoffatome und $R^1$, $R^2$, $R^3$ die in Anspruch 1 angegebene Bedeutung haben und daß $R^{10}$ Wasserstoff bedeutet.

7. Verbindungen gemäß einem der Ansprüche 2 bis 5 zur Verwendung als Heilmittel.

8. Mittel enthaltend eine Verbindung gemäß einem der Ansprüche 2 bis 5.

9. Verwendung einer Verbindung gemäß einem der Ansprüche 2 bis 5 als Heilmittel.